(19) Europäisches Patentamt
European Patent Office
Office européen des brevets

(11) Veröffentlichungsnummer: **0 002 477**
**B1**

(12) # EUROPÄISCHE PATENTSCHRIFT

(21) Anmeldenummer: **78101535.9**

(22) Anmeldetag: **04.12.78**

(51) Int. Cl.$^3$: **C 07 C 21/02,**
C 07 C 17/00

(54) **Verfahren zur Herstellung von 1,1-Dihalogen-4-methyl-1,3-pentadienen**

(30) Priorität: **16.12.77 DE 2756271**

(43) Veröffentlichungstag der Anmeldung:
**27.06.79 Patentblatt 79/13**

(45) Bekanntmachung des Hinweises auf die Patenterteilung:
**06.08.80 Patentblatt 80/16**

(84) Benannte Vertragsstaaten:
**BE CH DE FR GB IT NL**

(56) Entgegenhaltungen:
**FR - A - 2 321 468**

(73) Patentinhaber: **Bayer AG**
**Zentralbereich Patente, Marken und Lizenzen**
**D - 5090 Leverkusen 1, Bayerwerk (DE)**

(72) Erfinder: **Arlt, Dieter, Dr.**
**Rybniker Strasse 2**
**D - 5000 Köln 80 (DE)**
**Jautelat, Manfred, Dr.**
**Buchenweg 18**
**D - 5093 Burscheid 2 (DE)**

**EP 0 002 477 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Verfahren zur Herstellung von 1,1 - Dihalogen - 4 - methyl - 1,3 - pentadienen

Die Erfindung betrifft ein Verfahren zur Herstellung von 1,1 - Dihalogen - 4 - methyl 1,3 - pentadienen.

Es ist bekannt, 1,1 - Dichlor - 4 - methyl - 1,3 - pentadiene durch Umsetzung von 1,1,1 - Trichlor - 2 - acetoxy - 4 - methyl - 3 - penten mit Zinkstaub und Essigsäure herzustellen (Coll. Czech. Chem.Commun. *24* 2230 (1959)).

Es ist weiter bekannt, 1,1 - Dihalogen - 4 - methyl - pentadiene durch Umsetzung von Isobuten mit einem 1,1,2 - Trihalogenäthylen in der Dampfphase zu erhalten (DT-OS 2 629 868).

Weiter ist bekannt, durch elektrochemische Reduktion der isomeren 1,1,1 - Trihalogen - 2 - hydroxy - 4 - methyl - pentene zu den isomeren 1,1, - Dihalogen - 4 - methyl - pentadienen zu gelangen (US 4 022 672).

Weiter ist bekannt, 1,1 - Dihalogen - 4 - methyl - pentadiene durch Dehydratisierung und Dehydrohalogenierung von 1,1,1 - Trihalogen - 4 - methyl - 4 - hydroxypentanen herzustellen (FR-A 2 321 468).

Es wurde ein Verfahren zur Herstellung von 1,1 - Dihalogen - 4 - methyl - 1,3 - pentadienen gefunden, das dadurch gekennzeichnet ist, daß man 2,2 - Dimethyl - 3,5,5,5 - tetrahalogen - pentyl - 1 - Derivate der Formel

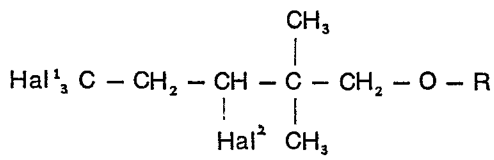

(I),

in der

$Hal^1$ und $Hal^2$ unabhängig voneinander gleiches oder verschiedenes Halogen bedeuten und

R für Wasserstoff oder $C_1$—$C_5$ - Acyl steht.

mit basisch reagierenden Stoffen im Temperaturbereich von etwa 20 bis etwa 160°C, gegebenenfalls in Gegenwart von inerten Verdünnungsmitteln, unsetzt.

Als Halogenetome $Hal^1$ und $Hal^2$ seien beispielsweise Fluor, Chlor, Brom und Jod, bevorzugt Fluor, Chlor und Brom, besonders bevorzugt Chlor und Brom, genannt.

Als Acyl-Reste (R) seien solche mit 1—5 Kohlenstoffatomen, beispielsweise Formyl, Acetyl, Propionyl, Butyryl, Isobuytyryl, Valeroyl und Isovaleroyl, genannt. Bevorzugt werden Acyl-Reste (R) mit 1 bis 3 Kohlenstoffenatomen, beispielsweise Formyl, Acetyl oder Propionyl. Besonders bevorzugt ist der Acetylrest.

Die 2,2 - Dimethyl - 3,5,5,5 - tetrahalogen - pentyl - 1 - Derivate der Formel (I) können aus den bekannten 2,2 - Dimethyl - 3 - buten - 1 - ol - Derivaten (J. Org. Chem. *30,* 10 (1965)) durch die ebenfalls bekannte Addition von Tetrahalogenmethanen in Gegenwart von Radikalbildnern (J.Am.Chem.Soc. *69,* 1100 (1947)) hergestellt werden.

Als Beispiele für erfindungsgemäß verwendbare Ausgangsstoffe seien im einzelnen genannt:
2,2-Dimethyl-3,5,5,5-tetrachlorpentylacetat,
2,2-Dimethyl-3,5,5,5-tetrachlorpentan-1-ol,
2,2-Dimethyl-3,5,5,5-tetrabrompentylacetat,
2,2-Dimethyl-3,5,5,5-tetrabrompentan-1-ol,
3-Brom-2,2-dimethyl-5,5,5-trichlorpentylacetat,
3-Brom-2,2-dimethyl-5,5,5-trichlorpentan-1-ol,
3-Chloro-2,2-dimethyl-5,5,5-trifluorpentylacetat,
3-Chlor-2,2-dimethyl-5,5,5-trifluorpentan-1-ol,
2,2-Dimethyl-3,5,5,5-tetrachlorpentylpropionat,
2,2-Dimethyl-3,5,5,5-tetrachlorpentylbutyrat,
2,2-Dimethyl-3,5,5,5-tetrachlorpentylvaleroat.

Basisch reagierende Stoffe für das erfindungsgemäße Verfahren können der Gruppe der tertiären Amine, der Alkalihydroxide, der Alkalicarbonate, der Alkoholate oder der Amide angehören. Als tertiäre Amine können beispielsweise Triäthylamin, Dimethyl-benzyl-amin, Pyridin, Äthyl-diisopropylamin, Dimethylanilin, 1,8-Diazabicyclo(5,4,0)-undec-7-en, 1,5-Diazabicyclo(4,3,0)-non-5-en, oder Triäthylendiamin eingesetzt werden. Auch Hydroxide tertiärer Amine können, beispielsweise durch Zugabe der entsprechenden, Menge Wasser zu einem der möglichen tertiären Amine, verwendet werden. Diese Verfahrensweise ist besonders deshalb vorteilhaft, weil sie es ermöglicht, Amine, die aus einer vorangegangenen Reinigungsstufe noch Wasser enthalten, ohne vorherige Trocknung einzusetzen.

Als Alkalihydroxide seien beispielsweise Natriumhydroxid oder Kaliumhydroxid genannt. Als alkalicarbonate seien beispielsweise Natriumcarbonat oder Kaliumcarbonat genannt. Als Alkoholate seien beispielsweise Natriummethylat, Natriumäthylat oder Kalium-tert.-butylat genannt. Als Amide seien beispielweise Natriumamid, Kaliumamid oder Lithiumdiisopropylamid genannt.

Bevorzugt werden Alkalihydroxide im erfindungsgemäßen Verfahren eingesetzt. Ganz besonders bevorzugt wird Natriumhydroxid eingesetzt.

Der Ausgangsstoff der Formel (I) und der basisch reagierende Stoff werden im molaren Verhältnis 1:2 bis 1:5, vorzugsweise 1:2,1 bis 1:3,5, eingesetzt.

Das erfindungsgemäße Verfahren kann mit oder ohne Verdünnungs mittel durchgeführt werden. Zweckmäßigerweise arbeitet man dann ohne Verdünnungsmittel, wenn die Ausgangsstoffe flüssig sind.

Als Verdünnungsmittel sind organische und anorganische Lösungsmittel geeignet, die unter den Reaktionsbedingungen inert sind. Inerte organische Lösungsmittel können der Gruppe der Kohlenwasserstoffe, beispielsweise Benzin, Cyclohexan oder Toluol, der halogenierten Kohlenwasserstoffe, beispielsweise Methylenchlorid, 1,2-Dichloräthan, Chlorbenzol oder Dichlorbenzol, der Äther, beispielsweise Diäthyläther, Tetrahydrofuran, Dioxan oder Dimethoxyäthan, oder der Alkohole, beispielsweise Methanol, Äthanol, Butanol oder Äthylenglykol, angehören. Als weitere inerte organische Lösungsmittel seien beispielsweise Acetonitril, Dimethylsulfoxid, Dimethylformamid oder Hexamethylphoshorsäuretriamid genannt.

Als inertes anorganisches Lösungsmittel sei beispielsweise das Wasser genannt.

Eine bevorzugte Verfahrensvariante ist die Verwendung wasserhaltiger Lösungsmittel bei gleichzeitiger Verwendung von Alkalihydroxiden als basisch reagierende Stoffe. Diese Variante hat den Vorteil, daß man organische Lösungsmittel, die mit Wasser mischbar sind, ohne vorherige Trocknung einsetzen kann.

Das erfindungsgemäße Verfahren kann am Beispiel der Umsetzung von 2,2-Dimethyl-3,5,5,5-tetrachlorpentylacetat mit Natriumäthylat zum 1,1-Dichlor-4-methyl-1,3-pentadien durch die folgende Formelgleichung erläutert werden:

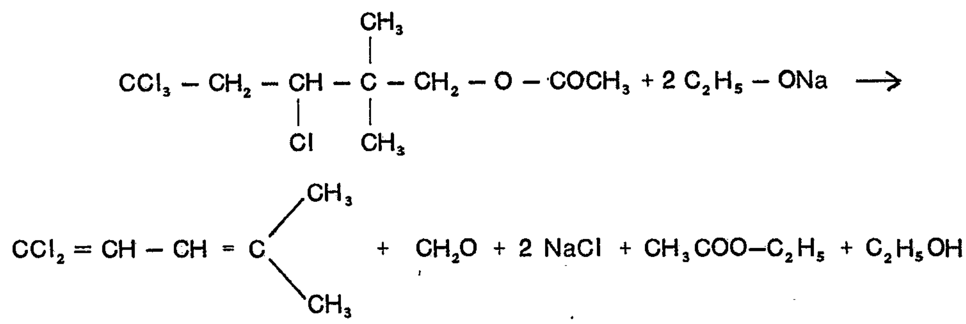

Mögliche Zwischenstufen des erfindungsgemäßen Verfahrens, die bei geeigneter Reaktionsführung zu isolieren sind, oder die auf andere Weise hergestellt werden können, können selbstverständlich im erfindungsgemäßen Verfahren anstelle der Ausgangsstoffe eingesetzt werden. Als Beispiele für solche mölichen Zwischenstufen seien die folgenden Verbindungen formelmäßig angeführt, wobei $Hal^1$, $Hal^2$ und R die weiter oben angegebene Bedeutung haben:

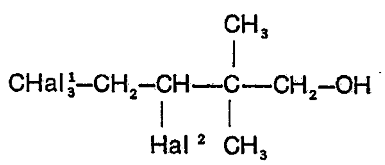 , 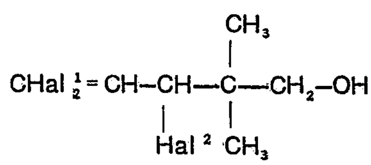 ,

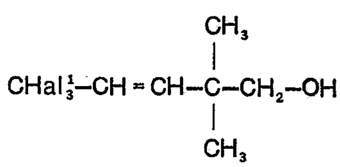 , 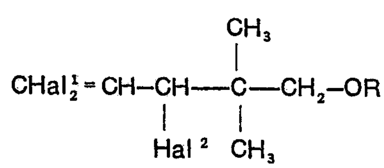 ,

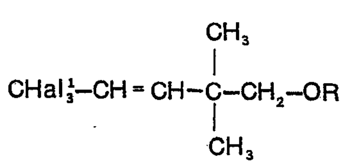

Das erfindungsgemäße Verfahren kann beispielsweise wie folgt durchgeführt werden:

Der basisch reagierende Stoff und gegebenenfalls das Verdünnungsmittel werden vorgelegt und der Ausgangsstoff der Formel (I) bei Raumtemperatur zugegeben. Anschließend wird das Reaktionsgemisch gerührt. Das Reaktionsgemisch wird durch Aufnehmen in einem geeigneten Lösungsmittel,

beispielsweise Pentan oder Methylenchlorid, anschließendem Waschen mit Wasser und nachfolgender Destillation aufgearbeitet.

Die nach dem erfindungsgemäßen Verfahren herstellbaren 1,1-Dihalogen-4-methyl-1,3-pentadiene der Formel

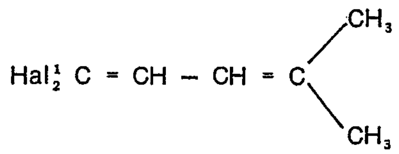

(II),

in der $Hal^1$ für Halogen steht,
sind bekannt und können als Zwischenprodukt zur Herstellung von Insektiziden, wie beispielsweise der 2-(2,2-Dichlorvinyl)-3,3-dimethyl-cyclopropancarbonsäure und ihrer Ester (Coll. Czech. Chem. Commun. *24*, 2230 (1959)) dienen.

Die zum Stand der Technik gehörenden und weiter oben beschriebenen Herstellungsverfahren für die Verbindungen der Formel (II) haben den Nachteil, daß sie teilweise über zahlreiche Reaktionsstufen verlaufen (Coll. Czech. Chem. Commun, *24*, 2230 (1959); DT—OS 2 629 868), teilweise liefern sie nur geringe Ausbeuten (US 4 022 672). Das erfindungsgemäße Verfahren vermeidet die angegebenen Nachteile, indem es aus einfach erhältlichen Vorprodukten in einer einstufigen Reaktion hohe Ausbeuten liefert.

Es ist als ausgesprochen überraschend zu bezeichnen, daß trotz der möglichen und weiter oben dargestellten Zwischenstufen, die teilweise nebeneinander, teilweise nacheinander auftreten können, das erfindungsgemäße Verfahren in einer sogenannten Eintopf-Reaktion in hoher Ausbeute zu dem Reaktionsprodukt der Formel (II) führt.

Beispiel 1 (Ausgangsprodukt)

16,5 g (0,116 mol) 2,2-Dimethyl-3-butenylacetat (J. Org. Chem. 30, 10 (1965)) werden in 120 ml Tetrachlormethan unter Zusatz von 3,6 g Dibenzoylperoxid 12 Stunden unter Rückfluß erhitzt und anschließend destilliert. Bei $Kp_{0,2}$ 96—99°C isoliert man 28,3 g (0,096 mol, 83 %) 2,2-Dimethyl-3,5,5,5-tetrachlorpentylacetat (Fp. 56—58°C).

Beispiel 2

29,6 (0,1 mol) 2,2-Dimethyl-3,5,5,5-tetrachlorpentylacetat werden bei Raumtemperatur in eine Lösung von 0,35 mol Natriumäthylat in 200 ml abs. Äthanol eingetropft und anschließend 2 Stunden unter Rückfluß erhitzt. Die Lösung wird mit n-Pentan verdünnt, filtriert und mit Wasser ausgewaschen. Die Pentanphase wird getrocknet, und das Pentan wird über eine Kolonne abdestilliert, Der Rückstand wird fraktioniert und liefert bei $Kp_{13}$ 57—59° 12,7 g (0,084 mol, 84 %) 1,1-Dichlor-4-methyl-1,3-pentadien.

Beispiel 3 (Ausgangsprodukt)

10 g (0,1 Mol) 2,2-Dimethyl-3-buten-1-ol werden in 100 ml Tetrachlormethan unter Zusatz von 2,4 g Dibenzoylperoxid 21 Stunden unter Rückfluß erhitzt. Durch Destillation erhält man bei $Kp_{0,1}$ 88—92° 23,2 g (0,091 mol, 91 %) 2,2-Dimethyl-3,5,5,5-tetrachlorpentan-1-ol.

Beispiel 4

25,4 g (0,1 mol) 2,2-Dimethyl-3,5,5,5-tetrachlorpentanol werden in 140 ml einer 1,5 n Natriumäthylatlösung in Äthanol 3 Stunden unter Rückfluß erhitzt. Die Aufarbeitung erfolgt analog dem Beispiel 2. Nach Destillation isoliert man 13,9 g (0,092 mol, 92 %) 1,1-Dichlor-4-methyl-1,3-pentadien ($Kp_{15}$:58—60°C).

Beispiel 5 (Ausgangs-produkt)

7,1 g (50 mmol)2,2-Dimethyl-3-butenylacetat und 16,6 g (50 mmol) Tetrabrommethan werden zusammen mit 0,5 g Dibenzoyl-peroxid 5 Stunden auf 90° erhitzt. Die Destillation bei $Kp_{0,1}$: 120—125°C liefert 17,3 g (36,5 mmol, 73%) 2,2-Dimethyl-3,5,5,5-tetrabrompentylacetat.

Beispiel 6

23,7 g (50 mmol) 2,2-Dimethyl-3,5,5,5-tetrabrompentylacetat werden mit 160 mmol Natriumäthylat in 150 ml Äthanol 6 Stunden unter Rückfluß erhitzt. Die Lösung wird mit Methylenchlorid verdünnt und mit Wasser ausgeschüttelt. Nach dem Trocknen wird die Lösung im Vakuum destilliert. Bei $Kp_{20}$: 89—92° gewinnt man 10,6 g (44 mmol, 88 %) 1,1-Dibrom-4-methyl-1,3-pentadien.

Beispiel 7

29,6 g (0,1 mol) 2,2-Dimethyl-3,5,5,5-tetrachlorpentylacetat werden in 100 ml Methanol mit 14 g (0,35 mol) Natriumhydroxid 6 Stunden unter Rückfluß erhitzt. Die Aufarbeitung analog Beispiel 2 ergibt 81 % 1,1-Dichlor-4-methyl-1,3-pentadien.

Beispiel 8

29,6 g (0,1 Mol) 2,2-Dimethyl-3,5,5,5-tetrachlorpentylacetat werden mit 12 g (0,3 mol) Natriumhydroxid in einem Gemisch aus 100 ml Wasser und 100 ml Dioxan 8 Stunden unter Rückfluß erhitzt. Man isoliert 78 % 1,1-Dichlor-4-methyl-1,3-pentadien.

Beispiel 9

14,8 g (50 mmol) 2,2-Dimethyl-3,5,5,5-tetrachlorpentylacetat werden mit 22,8 g (150 mmol) 1,8-Diazabicyclo(5.4.0)-undec-7-en (DBU) in 100 ml Äthanol 4 Stunden unter Rückfluß erhitzt. Die Lösung wird nach dem Erkalten mit Wasser verdünnt und mit n-Pentan extrahiert. Aus der organischen Phase werden nach Abdestillieren des Lösungsmittels durch weitere Destillation 5,6 g (37 mmol, das entspricht einer Ausbeute von 74 % der Theorie) 1,1-Dichlor-4-methyl-1,3-pentadien ($Kp_{20}$: 64—67°C) gewonnen.

**Patentansprüche**

1. Verfahren zur Herstellung von 1,1-Dihalogen-4-methyl-1,3-pentadienen der Formel

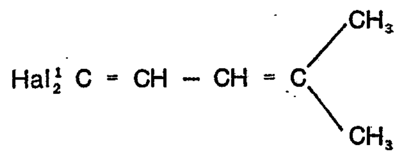

,

in der
$Hal^1$ für Halogen steht,
dadurch gekennzeihnet, daß man 2,2,-Dimethyl-3,5,5,5-tetrahalogen-pentyl-1-Derivate der Formel

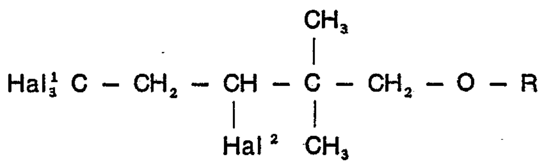

,

in der
$Hal^1$ und $Hal^2$ unabhängig voneinander gleiches oder verschiedenes Halogen bedeuten und
R fur Wasserstoff oder $C_1$—$C_5$-Acyl steht mit basisch reagierenden Stoffen im Temperaturbereich von etwa 20 bis etwa 160°C, gegebenenfalls in Gegenwart von inerten Verdünnungsmitteln, umsetzt.

2. Verfahren nach Anspruch 1, dadurch gekennzeichnet, daß man im Temperaturbereich von etwa 50 bis etwa 120°C arbeitet.

3. Verfahren nach Anspruch 1 und 2, dadurch gekennzeichnet, daß man als basisch reagierenden Stoff Natriumhydroxid einsetzt.

4. Verfahren nach Anspruch 1 bis 3, dadurch gekennzeichnet, daß man wasserhaltige inerte Verdünnungsmittel einsetzt.

**Revendications**

1. Procédé de fabrication de 1,1-dihalo-4-méthyl-1,3-pentadiènes de formule:

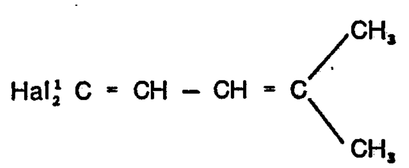

dans laquelle
$Hal^1$ représente de l'halogène,
caractérisé en ce qu'on fait réagir des dérivés de 2,2-diméthyl-3,5,5,5-tétrahalopentyle-1 de formule:

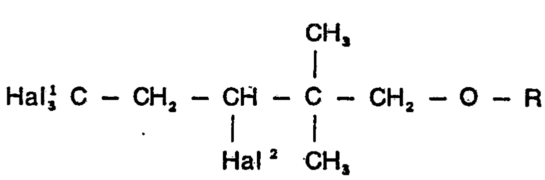

,

dans laquelle
$Hal^1$ et $Hal^2$ représentent indépendamment l'un de l'autre de l'halogène identique ou différent et
R représente de l'hydrogène ou un acyle en $C_1$—$C_5$,

avec des substances à réaction basique dans l'intervalle de température d'environ 20 à environ 160°C, éventuellement en présence de diluants inertes.

2. Procédé selon la revendication 1, caractérisé en ce qu'on opère dans l'intervalle de température d'environ 50 à environ 120°C.

3. Procédé selon l'une quelconque des revendications 1 et 2, caractérisé en ce qu'on utilise comme substance à réaction basique de l'hydroxyde de sodium.

4. Procédé selon l'une quelconque des revendications 1 à 3, caractérisé en ce qu'on utilise des diluants inertes aqueux.

**Claims**

1. Process for the preparation of 1,1-dihalogeno-4-methyl-1,3-pentadienes of the formula

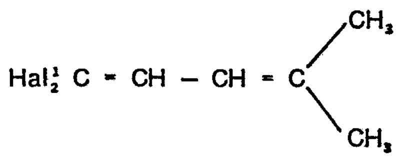

in which

Hal¹ represents halogen,

characterised in that 2,2-Dimethyl-3,5,5,5-tetrahalogenopent-1-yl derivatives of the formula

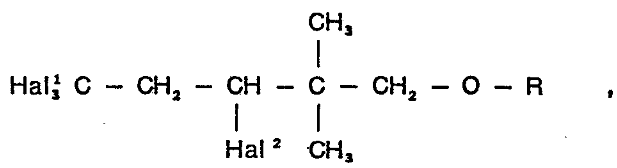

in which Hal¹ and Hal² independently of one another denote identical or different halogen, and

R represents hydrogen or $C_1$—$C_5$-acyl,

are reacted with substances having a basic reaction, in the temperature range from about 20° to about 160°C, optionally in the presence of inert diluents.

2. A process according to claim 1, characterised in that it is carried out in the temperature range from about 50° to about 120°C.

3. A process according to claim 1 and 2, characterised in that sodium hydroxide is employed as the substance having a basic reaction.

4. A process according to claim 1 to 3, characterised in that water-containing inert diluents are employed.